Europäisches Patentamt

(19) European Patent Office

. Office européen des brevets

(11) Publication number: **0 078 537**
**B1**

(12) **EUROPEAN PATENT SPECIFICATION**

(45) Date of publication of patent specification: **12.08.87**

(51) Int. Cl.⁴: **C 12 N 15/00, C 12 N 1/20** // **C12R1/15**

(21) Application number: **82110095.5**

(22) Date of filing: **02.11.82**

(54) **Plasmid pHM 1519.**

(30) Priority: **02.11.81 JP 176193/81**

(43) Date of publication of application:
**11.05.83 Bulletin 83/19**

(45) Publication of the grant of the patent:
**12.08.87 Bulletin 87/33**

(84) Designated Contracting States:
**DE FR GB**

(56) References cited:
**EP-A-0 058 889**
**EP-A-0 066 129**
**EP-A-0 071 023**

(73) Proprietor: **AJINOMOTO CO., INC.**
**5-8, Kyobashi 1-chome, Chuo-ku**
**Tokyo 104 (JP)**

(72) Inventor: **Matsui, Hiroshi**
**No. 1-19-16-101, Namiki Kanazawa-ku**
**Yokohama-shi Kanagawa-ken (JP)**
Inventor: **Miwa, Kiyoshi**
**3-304, Sagamidai-Jutaku No. 531, Iwase**
**Matsudo-shi Chiba-ken (JP)**
Inventor: **Terabe, Mahito**
**Tama Plaza Danchi 1-205 No. 1-4**
**Utsukushigaoka**
**Midori-ku Yokohama-shi Kanagawa-ken (JP)**
Inventor: **Nakamori, Shigeru**
**No. 2153-187, Kamariya-cho Kanazawa-ku**
**Yokohama-shi Kanagawa-ken (JP)**
Inventor: **Sano, Konosuke**
**No. 1-35-9, Uehara**
**Shibuya-ku Tokyo (JP)**

(74) Representative: **Schübel-Hopf, Ursula et al**
**Strehl, Schübel-Hopf, Schulz Patentanwälte**
**Widenmayerstrasse 17**
**D-8000 München 22 (DE)**

Courier Press, Leamington Spa, England.

### Description

The present invention relates to a plasmid, designated as plasmid pHM 1519 which is capable of propagating in *Coryneform* glutamic acid-producing bacteria.

*Coryneform* glutamic acid producing bacteria, so-called "*Coryneform* bacteria", are known to produce substantial amounts of L-glutamic acid, and mutants of the *Coryneform* glutamic acid producing bacteria produce amino acids such as lysine and purine nucleotides such as inosinic acid. Therefore, they are of great importance to the fermentation industry.

The recently developed gene splicing techniques can successfully be applied for producing or improving industrial microorganisms, especially in the case of *Escherichia coli*. It has been difficult, however, to apply the gene splicing techniques for producing or improving industrially useful microorganisms, which are *Coryneform* glutamic acid-producing bacteria, since suitable plasmids useful for the construction of such industrially useful *Coryneform* glutamic acid producing bacteria have not yet been developed.

Although a plasmid capable of propagating in *Coryneform* glutamic acid-producing bacteria was reported in *Agric. Biol. Chem., 43*, 867, (1979), the molecular weight of the known plasmid is 37 megadalton which is too large and inconvenient to use it for the construction of industrial microorganisms of *Coryneform* bacteria by gene splicing techniques.

Therefore a continuous need exists for the development of a novel plasmid useful for the construction of industrially useful *Coryneform* glutamic acid-producing bacteria.

It is accordingly one object of the present invention to provide a plasmid useful for the construction of industrially useful *Coryneform* glutamic acid-producing bacteria.

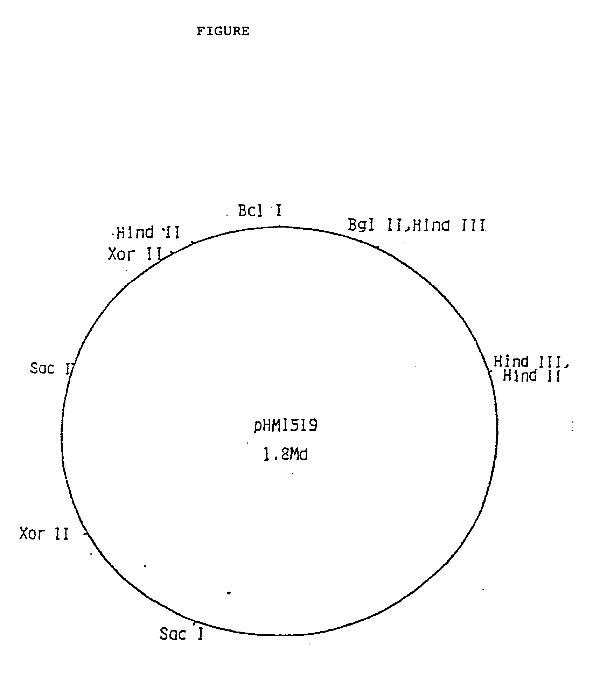

The above stated problems may be solved by a substantially pure plasmid pHM 1519, which is capable of propagating in a Coryneform glutamic acid producing bacterium as a host strain, which is characterized by a molecular weight of 1.8 megadalton and the restriction endonuclease-cleavage chart shown in the Figure and being obtainable from Corynebacterium glutaricum ATCC 13058.

The present invention further comprises the above described plasmid into which a foreign gene is inserted. The plasmid pHM 1519 may advantageously be used for transforming a Coryneform glutamic acid producing bacterium as a recipient.

A more complete appreciation of the invention and of many of the attendant advantages thereof will be readily obtained as the same becomes better understood by reference to the following detailed description when considered in connection with the accompanying drawings, wherein the Figure is the restriction endonuclease cleavage chart of the plasmid pHM 1519.

In the following preferred embodiments of the invention are described in more detail.

Plasmid pHM 1519 is separated from *Corynebacterium glutamicum* ATCC 13058. The plasmid has the following characteristics:

The molecular weights of the plasmid calculated by the migration distance on agarose gel electrophoresis and the length of the DNA-chains under an electron microscope are both 1.8 megadalton. The sensitivity to restriction enzymes and the restriction chart of each plasmid are the same as shown in Table 1 and in the Figure, respectively.

The plasmid pHM 1519 can be obtained from the cells of the deposited microorganisms *Corynebacterium glutamicum* ATCC 13058 by lysing the cells previously harvested at a late exponential growth phase by lysozyme and SDS, adding polyethylene glycol to the supernatant obtained from the lysate by centrifugation at 30,000 xg, and purifying the precipitated DNA by fractionation using cesium chloride-ethidium bromide equilibrium density gradient centrifugation.

# 0 078 537

TABLE 1

| | Restriction Enzyme | Number of Restriction Sites |
|---|---|---|
| Ava I | Anabena variabilis | $\geqq 4$ |
| Bcl I | Bacillus caldolyticus | 1 |
| BamH I | Bacillus amyloliquefaciens H | 0 |
| Bgl II | Bacillus globigii | 1 |
| EcoR I | Escherichia coli RI+ | 1 |
| Hae II | Haemophilus aegyptius | >7 |
| HgiA I | Herpetosiphon giganteus | $\geqq 4$ |
| Hind III | Haemophilus influenzae | 2 |
| Kpn I | Klebsiella pneumoniae | 0 |
| Sma I | Serratia marcescens | 0 |
| Sst I | Streptomyces stanford | 2 |
| Xba I | Xanthomonas badrii | 0 |
| Xho I | Xanthomonas holicola | 0 |
| Xma I | Xanthomonas malvacearum | 0 |

*Coryneform* bacteria are aerobic, gram-positive rods, non-sporulating, and non-acidfast, and are described in *Bergey's Manual of Determinative Bacteriology*, 8th ed., 599, (1974). Examples of wild strains of *Coryneform* glutamic acid-producing bacteria useful as hosts in this invention are as follows:

*Brevibacterium divaricatum* ATCC 14020,
*Brevibacterium saccharoliticum* ATCC 14066,
*Brevibacterium immariophilum* ATCC 14068,
*Brevibacterium lactofermentum* ATCC 13869,
*Brevibacterium roseum* ATCC 13825,
*Brevibacterium flavum* ATCC 13826,
*Brevibacterium thiogenitalis* ATCC 19240,
*Corynebacterium acetoacidophilum* ATCC 13870,
*Corynebacterium acetoglutamicum* ATCC 15806,
*Corynebacterium callunae* ATCC 15991,
*Corynebacterium glutamicum* ATCC 13032, 13060,
*Corynebacterium lilium* ATCC 15990,
*Corynebacterium melassecola* ATCC 17965,
*Microbacterium ammoniaphilum* ATCC 15354.

*Coryneform* glutamic acid-producing bacteria also include mutants which have lost the ability to produce glutamic acid or are capable of producing other amino acids such as lysine and arginine; purine nucleosides such as inosine; purine nucleotides such as inosine-5'-monophosphate; and other fermentation products.

In the cells of *Coryneform* glutamic acid-producing bacteria, the plasmids of the present invention are stably maintained. Since the plasmids of the present invention can propagate in cells of *Coryneform* glutamic acid-producing bacteria, the information of a foreign gene inserted in the plasmid can be amplified in the hosts.

The incorporation of plasmid DNA into the hosts of *Coryneform* glutamic acid-producing bacteria can be done by treating the cells of the DNA-recipient with calcium chloride to increase the permeability for DNA, as is reported regarding *E. coli* K—12 (Mandel, M and Higa, A., *J. Mol. Biol., 53*, 159 (1970), or by incorporation into the host at a specific stage of growth when the cells become capable of incorporating DNA therein (competent cells) as is reported for *Bacillus subtilis* (Duncan, C. H., Wilson, G. A., and Young, F. E., *Gene 1*, 153 (1977)).

3

The plasmid can also be incorporated into the DNA-recipient by forming protoplasts or spheroplasts of the DNA-recipient which easily incorporate plasmid DNA therein, as is known for *Bacillus subtilis*, actinomycetes and yeast (Chang, S. and Choen, S. N., *Molec. Gen. Genet., 168*, 111 (1979); Bibb, M. J., Ward, J. M. and Hopwood, O. A., *Nature, 274*, 398 (1978); Hinnen, A., Hicks, J. B. and Fink, G. R., *Proc. Nat'l Acad. Sci.*, USA *75*, 1929 (1978)).

A foreign gene can be inserted into the plasmid of the present invention at the positions cleaved by the restriction enzymes shown in Table 1 and more preferably at the cleavage position whose number is one. Genom DNA of *Coryneform* glutamic acid-producing bacteria is the most preferred of the foreign genes. A preferred vector can be obtained from the present plasmid by removing all or a part of the DNA regions other than the drive-unit (vehicle unit), since the molecular weight becomes small. The replication number of the present plasmid is sufficiently large and therefore is suitable for amplifying foreign genes.

Having generally described this invention, a further understanding can be obtained by reference to certain specific examples which are provided herein for purposes of illustration only and are not intended to be limiting unless otherwise specified.

### Example 1

Preparation of pHM 1519 DNA

*Corynebacterium glutamicum* ATCC 13058 which possessed pHM 1519 was cultured at 30°C in CMG medium at pH 7.2 containing 1 g/dl peptone, 1 g/dl yeast extract, 0.5 g/dl glucose and 0.5 g/dl NaCl until the late exponential growth phase was reached. Cells were harvested and lysed by treatment with lysozyme and SDS. The lysate was centrifuged to obtain a supernatant, which was added to polyethylene glycol, and the resulting mixture was evaporated to precipitate DNA. The precipitate was dissolved in Tris-EDTA-NaCl buffer (containing 20 mM Tris, 20 mM NaCl and 1 mM EDTA) at pH 8.0, and the solution was subjected to cesium chloride-ethidium bromide equilibrium density gradient centrifugation, whereby a circular plasmid was obtained. Thus, 51 µg of pHM 1519 plasmid DNA was obtained. This method is disclosed in Tamio Yamakawa (ed.), SEIKAGAKU JIKKEN KOZA, vol 2, 73, Tokyo-Kagaku-Donin Co. (1975). As such pHM 1519 can be obtained easily from ATCC 13058.

Agarose-gel electrophoresis of digested plasmid

Commercially available restriction enzymes sold by Bethesda Research Laboratory Co., Boehringer Mannheim Co., New England Biolab Co., Worthington Biochemical Co. were used. The digestion by restriction enzyme was carried out using an excessive amount of restriction enzyme, e.g. more than 3 times the amount required. The digestion reaction was carried out according to the directions by the distributors.

The DNA fragments were thereafter subjected to agarose-gel electrophoresis by the method shown in P. A. Sharp et al., Biochemistry *12*, 3055 (1973). The agarose concentration was 0.7 to 1%, the voltage was 5 to 20 v and electrophoresis was continued for 1 to 3 hours.

Molecular weight of pHM 1519

The molecular weight of pHM 1519 was calculated by its migration distance in the electrophoresis on agarose gel and by the length of its DNA-chain as determined under an electron microscope, pHM 1519 was cleaved with Bcl I which cut the plasmid at one point, and subjected to agarose-gel electrophoresis shown above. The molecular weight was calculated based on the mobility differences of the λ DNA-Hind III fragment produced by Bethesda Research Laboratory.

Determination of the replication number of pHM 1519

*Corynebacterium glutamicum* ATCC 13058 containing pHM 1519 was cultured at 30°C in 4 ml of CMG medium at pH 7.0 to obtain cells at the exponential growth phase. The cells harvested were lysed by lysozyme-SDS, and the DNAs in the lysate were separated by precipitation. The DNAs were thereafter subjected to 0.7% agarose-gel electrophoresis, and from the gel obtained a photograph under ultra-violet light was taken. The negative-film thus obtained was put on a densitometer, and the density ratio of plasmid DNA fraction to total DNA was calculated. The replication number was determined as 100 when chromosomal DNA of ATCC 13058 is supposed as $3 \times 10^9$ dalton.

### Claims

1. A substantially pure plasmid pHM 1519, which is capable of propagating in a Coryneform glutamic acid producing bacterium as a host strain, characterized by a molecular weight of 1.8 megadalton and the restriction endonuclease-cleavage chart shown in the Figure and being obtainable from Corynebacterium glutamicum ATCC 13058.

2. A substantially pure plasmid which is obtained by insertion of a foreign gene into the plasmid according to claim 1.

3. The use of the plasmid according to claim 1 for providing a cloning vector for a Coryneform glutamic acid producing bacterium.

4. The use of a plasmid according to claim 2 for transforming a Coryneform glutamic acid producing bacterium as a recipient.

# 0 078 537

## Patentansprüche

1. Im wesentliches reines Plasmid pHM 1519, das zur Vermehrung in einem coryneformen Glutaminsäure produzierenden Bakterium als Wirtsstamm befähigt ist, gekennzeichnet durch ein Molekulargewicht von 1,8 Megadalton und das in der Figur gezeigte Restriktionsendonuclease-Spaltschema, und welches aus Corynebacterium glutamicum ATCC 13058 erhältlich ist.

2. Im wesentlichen reines Plasmid, welches durch Einfügen eines fremden Gens in das Plasmid gemäß Anspruch 1 erhalten wird.

3. Verwendung des Plasmids nach Anspruch 1 zur Bildung eines Klonierungsvektors für ein coryneformes Glutaminsäure produzierendes Bakterium.

4. Verwendung eines Plasmids nach Anspruch 2 zum Transformieren eines coryneformen Glutaminsäure produzierenden Bakteriums als Empfängerorganismus.

## Revendications

1. Un plasmide pHM 1519 essentiellement pur, capable de propagation dans une bactérie corynéforme productrice d'acide glutamique comme souche hôte, caractérisé par un poids moléculaire de 1,8 mégadalton et le diagramme de clivage par les endonucléases de restriction illustré par la figure et pouvant être obtenu à partir de *Corynebacterium glutamicum* ATCC 13058.

2. Un plasmide essentiellement pur obtenu par insertion d'un gène étranger dans le plasmide selon la revendication 1.

3. L'emploi du plasmide selon la revendication 1 pour l'obtention d'un vecteur de clonage pour une bactérie corynéforme productrice d'acide glutamique.

4. L'emploi d'un plasmide selon la revendication 2 pour la transformation d'une bactérie coryneform productrice d'acide glutamique servant de receveur.

5

FIGURE

pHM1519
1.8Md

Bcl I
Hind II
Xor II
BgI II, Hind III
Sac I
Hind III, Hind II
Xor II
Sac I